# FASCICULE DE BREVET EUROPEEN

(11) **EP 2 324 780 B1**
(45) Date de publication et mention de la délivrance du brevet: **13.02.2013**
(21) Numéro de dépôt: 10191950.4
(22) Date de dépôt: 19.11.2010
(51) Int. Cl.: A61B 17/17

(54) **Ensemble chirurgical d'assistance à l'implantation d'un composant glénoïdien de prothèse d'épaule**
Chirurgisches Set zur Unterstützung der Implantierung einer Schultergelenkspfannenprothese
surgical kit for assisting implantation of a glenoid component of a shoulder prosthesis

(30) Priorité: 15.01.2010 FR 1050267; 24.11.2009 US 263985 P
(43) Date de publication de la demande: 25.05.2011
(73) Titulaire: Tornier, 38330 Montbonnot-Saint-Martin (FR)
(72) Inventeur: Ball, Robert J., WEST OLIVE (Michigan), MI 49460 (US)
(74) Mandataire: Grand, Guillaume

(56) Documents cités:
- EP-A1- 1 457 159
- EP-A2- 0 903 127
- US-A- 5 030 219

## Description

La présente invention concerne un ensemble chirurgical d'assistance à l'implantation d'un composant glénoïdien de prothèse d'épaule sur un patient.

Le remplacement de la surface articulaire glénoïdienne de l'omoplate d'un être humain par un composant prothétique glénoïdien d'une prothèse d'épaule est une opération chirurgicale délicate. On constate que, selon la position d'implantation de ce composant glénoïdien, des risques de descellement du composant existent, en raison de la modification d'efforts appliqués à ce composant lors des mouvements ultérieurs de l'épaule prothésée. Actuellement, les chirurgiens orthopédiques choisissent un tel composant glénoïdien parmi une gamme d'implants, présentant des géométries, notamment des tailles, légèrement différentes les unes des autres, puis estiment empiriquement la position de l'implantation du composant glénoïdien choisi, notamment en appréciant visuellement la géométrie de la surface glénoïdienne du patient à opérer. Le chirurgien essaie, de manière peropératoire, de choisir et d'implanter le composant prothétique sur l'omoplate de manière à ce que, en service, ce composant reproduise la cinématique de la surface articulaire glénoïdienne d'origine du patient, tout en étant fixée de manière ferme et stable à la glène. Dans ce contexte, EP-A-1 457 159, sur lequel est basé le préambule de la revendication 1, propose un ancillaire pour guider de manière ajustable l'application d'un organe de perçage sur la glène d'un patient.

Une autre approche de cette problématique consiste, sur la base de données de cartographie pré-opératoires relatives à la glène du patient, à fabriquer un composant glénoïdien « sur mesure », c'est-à-dire spécifiquement personnalisé pour chaque patient opéré. Cette personnalisation concerne en particulier les aménagements du composant visant à le solidariser de manière pérenne à la glène, en tenant compte des caractéristiques précises de cette glène, propres au patient. Ce genre de solution « sur mesure » est évidemment très onéreux et son exploitation se révèle contraignante puisque des autorisations sanitaires sont nécessaires avant d'implanter le composant dans la mesure où ce dernier ne correspond pas à des spécifications standard qui auraient été préalablement admises par les Autorités sanitaires compétentes.

Le but de la présente invention est de proposer au chirurgien des moyens simples et faciles à utiliser pour l'aider à optimiser la position d'implantation d'un composant glénoïdien « standard ».

A cet effet, l'invention a pour objet un ensemble chirurgical d'assistance à l'implantation d'un composant glénoïdien de prothèse d'épaule sur un patient, tel que défini à la revendication 1.

L'idée à la base de l'invention est de mettre à la disposition du chirurgien des moyens simples et intuitifs pour réaliser, pour chaque patient opéré, une implantation « sur mesure » d'un composant glénoïdien « standard ». Pour ce faire, l'ensemble conforme à l'invention comporte au moins un ancillaire dont la platine, destinée à être appuyée de manière ajustée sur la glène du patient, est pourvue de moyens de guidage tels que soit plusieurs éléments fixes de guidage de l'outil de préparation de la glène, par exemple des trous de guidage d'un foret de perçage de la glène, soit un organe mobile de guidage d'un tel outil, déplaçable entre des positions de réglage, soit à la fois de tels éléments de guidage fixes et un tel organe de guidage mobile. Lors de l'intervention chirurgicale proprement dite, le chirurgien devra choisir soit l'un de ces éléments de guidage fixes, soit l'une des positions de réglage de l'organe de guidage mobile, pour appliquer l'outil de préparation précité. Selon l'invention, le choix du chirurgien est aidé par des représentations graphiques de, à la fois, la glène du patient et un composant glénoïdien fictivement implanté sur cette glène. Ces représentations graphiques sont aussi nombreuses que les différentes configurations d'application de l'outil fournies par les moyens de guidage, c'est-à-dire typiquement aussi nombreuses que les éléments de guidage fixes ou que les positions de réglage de l'organe de guidage mobile, en étant respectivement associées à ces derniers de manière univoque selon une relation prédictive. Ainsi, grâce à la représentation graphique associée à un premier de ces éléments de guidage ou à une première de ces positions de réglage, le chirurgien peut visualiser directement et facilement la position dans laquelle sera implanté le composant glénoïdien du patient dans l'hypothèse où le chirurgien guiderait l'application de l'outil de préparation par ce premier des éléments de guidage ou par l'organe mobile placé dans cette première des positions de réglage. De même, grâce à la représentation associée à un second élément de guidage ou à une seconde position de réglage, le chirurgien visualise la position dans laquelle serait implanté le composant glénoïdien sur la glène dans l'hypothèse où le chirurgien guiderait l'outil de préparation par ce second élément de guidage. Et ainsi de suite pour les différentes autres représentations graphiques. En pratique, les différentes représentations graphiques et les éléments de guidage ou positions de réglage correspondants sont ainsi prévus pour ajuster la position d'implantation relative entre la glène et le composant glénoïdien selon un ou plusieurs degrés de liberté sur lesquels le chirurgien a avantageusement l'habitude de jouer pour optimiser l'implantation.

Autrement dit, la ou les séries de représentation graphiques, respectivement associées à le ou aux différents degrés de liberté envisagés pour l'implantation, fournissent au chirurgien les différentes possibilités de résultat pour l'implantation du composant glénoïdien sur la glène spécifique du patient qui va être opéré, selon que le chirurgien utilise tel ou tel élément de guidage ou bien telle ou telle position de réglage pour préparer la glène à recevoir le composant glénoïdien. Visuellement, le chirurgien apprécie, éventuellement en pré-opératoire, la situation médicale propre à ce patient et décide de la configuration d'implantation qu'il estime la meilleure. Grâce à l'identification de la représentation graphique correspondant à son choix, il sait quel élément de guidage fixe ou quelle position de réglage de l'organe mobile il utilisera en peropératoire pour appliquer l'outil de préparation osseuse de la glène en vue de réaliser effectivement la pose du composant glénoïdien.

Par ailleurs, un autre avantage de l'ensemble chirurgical conforme à l'invention est lié au fait que les représentations graphiques évoquées ci-dessus n'ont pas nécessairement à être réalisées par le chirurgien ou un soignant ayant des compétences chirurgicales similaires, mais, au contraire, ces représentations graphiques peuvent être obtenues, avantageusement de manière pré-opératoire, par un opérateur moins qualifié, à partir de données de cartographie pré-opératoires relatives à la glène du patient à opérer et de données dimensionnelles préétablies relatives au composant glénoïdien à implanter, le cas échéant avec l'assistance d'un ordinateur, sans nécessiter pour autant de moyens logiciels sophistiqués. De plus, grâce à ces représentations graphiques, le chirurgien peut ajuster facilement sa stratégie d'intervention, le cas échéant juste avant l'intervention.

D'autres caractéristiques avantageuses de l'ensemble chirurgical conforme à l'invention, prises isolément ou selon toutes les combinaisons techniquement possibles, sont spécifiées aux revendications dépendantes 2 à 10.

En lien avec l'ensemble selon l'invention, il est proposé une première méthode chirurgicale d'implantation d'un composant glénoïdien de prothèse d'épaule sur la glène d'un patient, dans laquelle :
- on dispose de données dimensionnelles préétablies relatives au composant glénoïdien ;
- on recueille des données de cartographie pré-opératoires relatives à la glène du patient ;
- à partir des données de cartographie pré-opératoires relatives à la glène et des données dimensionnelles préétablies relatives au composant glénoïdien, on détermine par calcul au moins une série de plusieurs positions d'implantation fictive du composant glénoïdien sur la glène, qui sont réparties selon un degré de liberté prédéfini pour la ou chaque série ;

- pour chacune des positions d'implantation déterminées, on fournit une représentation graphique correspondante de, à la fois, la glène du patient et le composant glénoïdien fictivement implanté sur cette glène ;
- après avoir choisi l'une des représentations graphiques fournies, on appuie sur la glène du patient une platine d'ancillaire pourvue d'éléments fixes de guidage d'un outil de préparation osseuse, chacun de ces éléments de guidage étant associé de manière univoque à l'une des représentations graphiques, puis on applique l'outil de préparation osseuse à la glène, en guidant cet outil par l'élément de guidage fixe qui est associé à la représentation graphique choisie ; et
- on met en place le composant glénoïdien sur la glène préparée par l'outil.

Egalement en lien avec l'ensemble selon l'invention, il est proposé une seconde méthode chirurgicale d'implantation d'un composant glénoïdien de prothèse d'épaule sur la glène d'un patient, dans laquelle :
- on dispose de données dimensionnelles préétablies relatives au composant glénoïdien ;
- on recueille des données de cartographie pré-opératoires relatives à la glène du patient ;
- à partir des données de cartographie pré-opératoires relatives à la glène et des données dimensionnelles préétablies relatives au composant glénoïdien, on détermine par calcul au moins une série de plusieurs positions d'implantation fictive du composant glénoïdien sur la glène, qui sont réparties selon un degré de liberté prédéfini pour la ou chaque série ;
- pour chacune des positions d'implantation déterminées, on fournit une représentation graphique correspondante de, à la fois, la glène du patient et le composant glénoïdien fictivement implanté sur cette glène ;
- après avoir choisi l'une des représentations graphiques fournies, on appuie sur la glène du patient une platine d'ancillaire pourvue d'un organe mobile de guidage d'un outil de préparation osseuse, cet organe de guidage mobile étant déplaçable par rapport à la platine entre des positions de réglage dont chacune est associée de manière univoque à l'une des représentations graphiques, puis on applique l'outil de préparation osseuse à la glène, en guidant cet outil par l'organe de guidage mobile après avoir placé ce dernier dans sa position de réglage associée à la représentation graphique choisie ; et
- on met en place le composant glénoïdien sur la glène préparée par l'outil.

L'invention sera mieux comprise à la lecture de la description qui va suivre, donnée uniquement à titre d'exemple et faite en référence aux dessins sur lesquels :
- les figures 1 et 2 sont des coupes schématiques dans un plan frontal d'un patient à opérer, la figure 1 montrant un composant glénoïdien à implanter tandis que la figure 2 montre la glène du patient à prothéser ;
- la figure 3 est une vue analogue à la figure 2, montrant l'utilisation d'un ancillaire appartenant à un ensemble conforme à l'invention ;
- la figure 4 est une vue en élévation selon la flèche lV de la figure 3, et
- la figure 5 est un schéma montrant une série de représentations graphiques, appartenant à l'ensemble chirurgical conforme à l'invention.

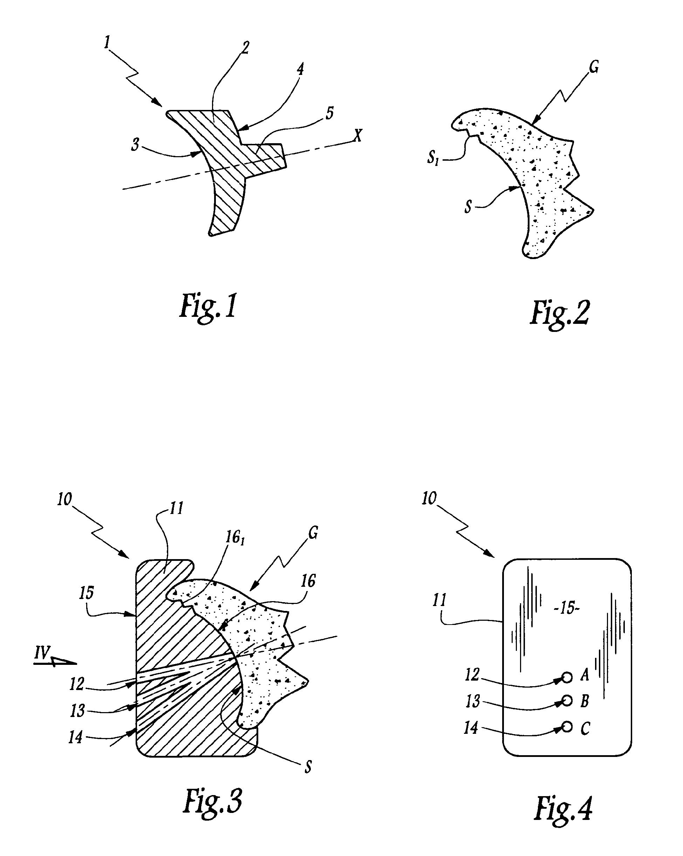

Sur la figure 1 est représenté un composant glénoïdien 1 d'une prothèse d'épaule, qui comporte un corps d'implant 2 présentant une forme globale de portion de cupule. Le corps d'implant 2 délimite ainsi deux faces principales opposées, à savoir une face articulaire 3 et une face d'appui 4. Plus précisément, la face articulaire 3 est conformée, au moins en partie, pour s'articuler contre une surface sensiblement complémentaire, non représentée sur les figures, délimitée soit par l'extrémité supérieure anatomique d'un humérus, soit par un composant huméral de la prothèse d'épaule précitée. Dans l'exemple montré sur la figure 1, cette face articulaire 3 est concave mais, en variante non représentée, elle peut tout aussi bien être convexe, la prothèse d'épaule correspondante étant alors généralement qualifiée de prothèse inversée. La face d'appui 4 est, quant à elle, destinée à être appuyée, directement ou indirectement, contre l'extrémité latérale osseuse d'une omoplate, autrement dit contre la glène de cet omoplate, préalablement préparée à cette fin. De manière connue en soi, cette face d'appui 4 est munie de moyens d'ancrage osseux dans la glène : dans l'exemple de réalisation considéré sur les figures, ces moyens d'ancrage se présentent sous la forme d'une quille saillante 5.

On souligne ici que le composant glénoïdien 1 considéré sur la figure 1 n'est qu'un exemple illustratif, montré de manière très schématique sur les figures. La présente invention s'applique à des composants glénoïdiens réalisés sous des formes très diverses. En particulier, le corps d'implant 2 peut aussi bien être monobloc qu'être constitué de l'assemblage de plusieurs parties, étant remarqué qu'un ou plusieurs matériaux, aussi bien de nature métallique que plastique, céramique ou pyrocarbone, peuvent être utilisés pour fabriquer ce corps. De même, d'autres formes que la quille 5 sont envisageables pour les moyens d'ancrage osseux dont est pourvue la face d'appui 4 du corps d'implant 2. On remarquera à ce propos que, selon la forme des moyens d'ancrage précités, la préparation de la glène sur laquelle le composant est à installer doit être adaptée, étant souligné que c'est justement la qualité de cette préparation qui déterminera le positionnement précis d'implantation du composant 1 vis-à-vis de la glène.

Sur la figure 2, est représentée la glène G de l'omoplate d'un patient. Cette glène G est montrée dans sa configuration anatomique : la glène G délimite ainsi une surface glénoïdienne S essentiellement concave, contre laquelle, normalement, vient s'articuler la tête anatomique de l'humérus du patient, non représentée, pour constituer l'articulation de l'épaule. Toutefois, on considère ici que la glène G du patient est à prothéser pour des raisons pathologiques ou accidentelles, c'est-à-dire que cette glène doit être munie d'un composant glénoïdien prothétique, comme expliqué en détail ci-après.

Pour illustrer la présente invention, on va décrire ci-dessous comment un chirurgien va implanter le composant glénoïdien 1 de la figure 1 sur la glène G de la figure 2, étant rappelé que l'une des difficultés de ce type d'intervention chirurgicale réside dans le choix et la réalisation d'une configuration d'implantation « optimale », c'est-à-dire une configuration d'implantation préférentielle aux yeux du chirurgien, sur la base de l'ensemble des paramètres médicaux portés à sa connaissance.

Pour bien comprendre l'invention, la description donnée ci-après, en regard des figures 3 à 5, s'intéresse spécifiquement à l'ajustement par le chirurgien de l'inclinaison du composant glénoïdien par rapport à la glène G dans le plan frontal du patient correspondant au plan de coupe des figures 1 et 2. Pour la forme de réalisation prise à titre d'exemple pour le composant glénoïdien 1, notamment avec sa quille 5, cela revient à s'intéresser à l'ajustement de l'inclinaison, dans le plan précité, de l'axe principal longitudinal X de la quille 5, cette inclinaison étant obtenue vis-à-vis de la glène G par la réalisation d'un canal de réception de la quille 5, que le chirurgien doit creuser dans la glène G à l'aide du foret d'une perceuse à usage médical, non représenté sur les figures.

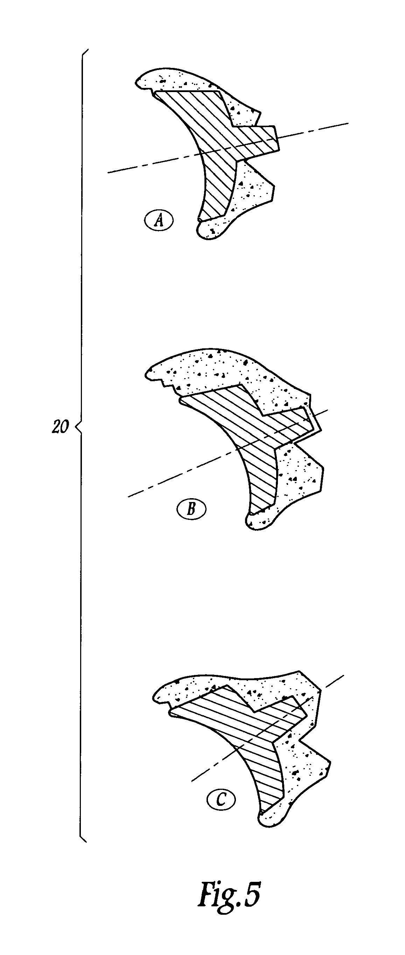

Dans le contexte d'utilisation qui vient d'être présenté juste ci-dessus, le chirurgien dispose, d'une part, d'un ancillaire 10, montré sur les figures 3 et 4, pour guider l'application du foret de perçage précité et, d'autre part, d'une série 20 de trois représentations graphiques, montrée sur la figure 5.

L'ancillaire 10 comprend une platine 11 traversée, de part en part, par trois trous distincts 12, 13 et 14, conformés chacun pour guider le foret de perçage précité, la section tranversale de chacun de ces trous étant sensiblement ajustée sur celle de ce foret. Les trous 12, 13 et 14 relient ainsi deux faces opposées de la platine 11, à savoir une face proximale 15, qui, en service, est tournée vers le chirurgien, et une face distale 16 qui est conformée de manière ajustée à la surface glénoïdienne S de la glène G du patient et qui, en service, est appuyée contre cette glène. Dans l'exemple de réalisation considéré sur les figures, les axes centraux des trous 12, 13 et 14 appartiennent à un même plan qui, lorsque la platine 11 est appuyée sur la glène G, correspond au plan frontal du patient, définissant le plan de coupe des figures 1 et 2. De plus, les axes de ces trous sont sensiblement concourants en un point appartenant à la face distale 16 : autrement dit, en s'éloignant de la face proximale 15, les trous 12, 13 et 14 convergent les uns vers les autres, jusqu'à déboucher sur la face distale 16 sous la forme d'un orifice commun. On comprend donc que, en fonction du trou utilisé parmi les trous 12, 13 et 14 pour guider le foret de perçage précité, le canal ainsi creusé dans la glène sera plus ou moins incliné dans le plan frontal du patient.

Les trous 12, 13 et 14 sont identifiés de manière visuelle sur la face proximale 15 de l'ancillaire 10 : dans l'exemple montré à la figure 4, les débouchés des trous 12, 13 et 14 sur la face 15 sont repérés par des lettres, à savoir respectivement «A », « B » et « C », inscrites sur la face 15.

Comme montré sur la figure 5, chaque représentation graphique de la série 20 correspond à un dessin montrant, à la fois, la glène G du patient opéré et le composant glénoïdien 1 fictivement implanté sur cette glène, la glène et le composant glénoïdien étant dessinés dans le plan frontal du patient correspondant au plan de coupe des figures 1 et 2. Les trois représentations graphiques de la série 20 se distinguent les unes des autres par le fait que le composant glénoïdien 1 est implanté fictivement sur la glène G dans trois positions différentes : dans l'exemple envisagé ici, les trois positions d'implantation diffèrent par l'inclinaison de l'axe X dans le plan frontal précité.

Selon l'invention, il est prévu une relation prédictive entre la série de représentations graphiques 20 et l'ancillaire 10. En effet, l'inclinaison d'implantation montrée par la représentation graphique la plus haute sur la figure 5 sera obtenue par le chirurgien si ce dernier utilise le trou 12 pour creuser le canal de réception de la quille 5, ce qui explique que cette représentation graphique est repérée par la lettre « A » qui est la lettre utilisée pour repérer le trou 12 sur la platine 11 de l'ancillaire. De même, la représentation graphique la plus basse sur la figure 5 montre l'inclinaison d'implantation du composant 1 sur la glène G dans l'hypothèse où c'est le trou de guidage 14 qui est utilisé, ce qui explique que cette représentation graphique est repérée par la lettre « C » utilisée pour repérer le trou 14. Bien entendu, une relation prédictive de la même teneur existe entre le trou de guidage 13 et la représentation graphique intermédiaire entre les représentations graphiques « A » et « C », moyennant l'utilisation de la lettre de repérage «B».

Pour bien comprendre la relation prédictive évoquée ci-dessus, on va maintenant décrire comment l'ancillaire 10 et la série de représentations graphiques 20 sont obtenus puis utilisés en vue d'implanter le composant glénoïdien 1 sur la glène G du patient.

Préalablement à l'intervention chirurgicale d'implantation proprement dite, on recueille des données de cartographie relatives à la glène G du patient à opérer. En pratique, ces données de cartographie pré-opératoires peuvent être obtenues de diverses manières. A titre d'exemple, des images scanners de la glène G sont utilisées : en particulier, dans l'exemple de mise en oeuvre envisagée ici, on réalise au moins une image scanner de la glène G dans le plan frontal correspondant au plan de coupe de la figure 2.

Par ailleurs, toujours en pré-opératoire, on dispose de données dimensionnelles relatives au composant glénoïdien 1. En pratique, ces données dimensionnelles sont généralement mises à disposition par le fabricant du composant glénoïdien. A défaut, elles peuvent être récupérées par des mesures appropriées.

Toujours en pré-opératoire, on utilise les données de cartographie pré-opératoires relatives à la glène G et les données dimensionnelles préétablies relatives au composant glénoïdien 1 pour déterminer, par calcul, plusieurs positions d'implantation fictives, en jouant au moins sur un degré de liberté prédéfini. Dans le cas de mise en oeuvre envisagé en regard des figures, cela revient à déterminer les trois positions d'inclinaison montrées par les représentations graphiques de la série 20. En pratique, il est intéressant de jouer sur l'inclinaison d'implantation en répartissant de manière prédéterminée les différentes positions d'implantation fictives : par exemple, l'inclinaison de l'axe X varie avec un pas valant un degré entre les différentes représentations graphiques.

Avantageusement, on remarquera que le procédé, décrit ci-dessus, pour obtenir la série de représentations graphiques 20 peut être mis en oeuvre par un opérateur n'ayant que peu, voire pas de connaissances médicales.

Parallèlement à l'obtention de la série de représentations graphiques, l'ancillaire 10 est fabriqué de façon à corréler de manière univoque ses trous de guidage 12, 13 et 14 et les trois représentations graphiques de la série 20. Pour ce faire, les trous 12, 13 et 14 sont conçus pour, lorsque la platine 11 est appuyée sur la surface glénoïdienne S de la glène G, appliquer un foret de perçage selon trois configurations différentes, plus précisément guider ce foret selon des directions respectives qui correspondent aux inclinaisons des trois représentations graphiques de la série 20. Comme expliqué plus haut, ces trous de guidage 12, 13 et 14 sont respectivement associés de manière univoque aux représentations graphiques repérées par les lettres « A », «B» et « C ».

On remarquera que la corrélation prédictive entre les trous 12, 13 et 14 et les trois représentations graphiques de la série 20 passe par un positionnement précis prédéterminé de la platine 11 sur la glène G lors de l'utilisation de l'ancillaire 10. La conformation ajustée de la face distale 16 vis-à-vis de la surface glénoïdienne S de la glène G contribue à ce positionnement précis, voire, avantageusement, assure exclusivement ce positionnement à condition de personnaliser spécifiquement au patient la conformation de cette face distale 16 de manière à n'autoriser qu'une seule configuration de coopération mécanique entre la platine 11 et la glène G : cela implique un ajustement rigoureux de la face 16 vis-à-vis de la surface glénoïdienne S, notamment en utilisant les données de cartographie pré-opératoires relatives à la glène G pour fabriquer la platine 11. En pratique, la face distale 16 de la platine 11 présente alors des reliefs personnalisés spécifiquement au patient opéré : par exemple, on voit sur la figure 3 que des reliefs 16₁ sont ajutés rigoureusement sur une zone altérée S₁ située dans la partie haute de la surface glénoïdienne S.

D'autres solutions sont envisageables pour repérer avec précision le positionnement de la platine 11 sur la glène G lors de l'utilisation de l'ancillaire 10. Par exemple, des systèmes gradués mécaniques ou des systèmes de repérage optiques ultrasoniques et/ou électromagnétiques sont envisageables.

Lors de l'intervention chirurgicale d'implantation proprement dite, le chirurgien prend connaissance visuellement de la série de représentations graphiques 20. En pratique, à cette fin, ces représentations graphiques lui sont fournies sur un support d'imprimerie, par exemple sur des feuilles de papier, ou bien lui sont affichées sur des écrans vidéo. Dans tous les cas, le chirurgien choisit à discrétion, parmi les représentations graphiques de la série 20, celle qu'il estime être la plus appropriée pour le patient opéré. Il prend alors connaissance de la lettre d'identification de cette représentation graphique, parmi les lettres « A », « B » et « C ». L'intervention chirurgicale se poursuit et, après avoir incisé les parties molles entourant l'épaule du patient, le chirurgien appuie la face distale 16 de la platine 11 de l'ancillaire 10 sur la surface glénoïdienne S de la glène G du patient, jusqu'à positionner la platine 11 convenablement contre la glène G. Le chirurgien creuse alors la glène G à l'aide d'un foret de perçage guidé par le trou, parmi les trous 12, 13 et 14, qui est associé à la représentation graphique que le chirurgien a préalablement choisi parmi la série 20. En pratique, le chirurgien identifie le trou pertinent grâce à la lettre d'identification correspond à la représentation graphique précitée.

L'intervention chirurgicale s'achève par la mise en place du composant glénoïdien 1 sur la glène G, en introduisant sa quille 5 dans le canal que le chirurgien vient de creuser dans la glène.

Divers aménagements et variantes à l'ancillaire 10, à la série de représentations graphiques 20 et à la méthode d'implantation décrits ci-dessus sont par ailleurs envisageables. A titre d'exemples :
- au moins un autre degré de liberté, que l'inclinaison dans un plan frontal au patient, peut être envisagé pour les positions d'implantation montrées par les représentations graphiques de la série 20, et ce aussi bien individuellement que combinés les uns aux autres ; c'est par exemple le cas pour la déviation antéropostérieure du composant glénoïdien 1 par rapport à la glène G dans un plan transversal au patient, pour le positionnement en hauteur dans le plan frontal, et pour l'angle de version glénoïdienne ; lorsque plusieurs degrés de liberté sont ainsi pris en considération, les représentations graphiques des positions d'implantation selon ces différents degrés de liberté sont présentées en autant de séries respectives, de manière à avantageusement former visuellement une matrice de représentations graphiques ;
- comme évoqué plus haut, l'ancrage du composant glénoïdien 1 de la glène G peut être réalisé par d'autres moyens que la quille 5, ce qui induit nécessairement une préparation osseuse différente pour la glène par le biais d'outils éventuellement différents que le foret de perçage évoqué jusqu'ici, tels que des broches ou des lames de scie, ce qui induit donc également, pour les éléments de guidage de ce ou ces autres outils, d'autres formes de réalisation possibles que les trous de guidage 12, 13 et 14, telles que des trous non cylindriques ou des fentes ;
- plutôt que de prévoir des éléments de guidage fixes sur la platine 11, tels que les trous 12, 13 et 14, on peut rapporter sur la platine un organe de guidage mobile ; cet organe est ainsi déplaçable sur la platine entre une succession de positions de réglage, dans chacune desquelles l'organe guide l'application de l'outil de préparation osseuse selon une configuration spécifique ; ainsi, d'un point de vue fonctionnel, les différentes positions dans lesquelles cet organe de guidage peut être réglé sont équivalentes à la succession des éléments de guidage fixes, tels les trous 12, 13 et 14 ; en pratique, les différentes positions de réglage de l'organe mobile peuvent être facilement repérées sur la platine 16, en prévoyant qu'elles soient réparties suivant une trajectoire graduée soit à l'aide de lettres d'identification, telles que les lettres « A », « B » et « C » décrites plus haut, soit à l'aide d'un marquage métrique à condition que les représentations graphiques respectivement associées de manière univoque aux différentes positions de réglage incluent une information métrique correspondant à ce marquage ;
- les représentations graphiques de la série ou chaque 20 peuvent inclure chacune plusieurs dessins, en particulier sous des angles de vue différents, ce qui s'avère utile, voire indispensable lorsque plusieurs degrés de liberté sont pris en considération pour positionner l'implantation du composant glénoïdien 1 à l'aide de l'invention ;
- de même, les représentations graphiques de la ou chaque série 20 peuvent inclure chacune des valeurs prédictives, résultantes de la position d'implantation montrée sur la représentation graphique ; ces valeurs peuvent être, à titre d'exemples non limitatifs, la mobilité articulaire rendue possible par la position d'implantation montrée, la quantification du contact articulaire en ce qui concerne son étendue ou les contraintes transmises, la quantification de l'usure prothétique, etc. ; ces diverses valeurs de caractéristiques mécaniques liées à l'implantation du composant glénoïdien sont notamment obtenues grâce à une simulation numérique de l'articulation virtuellement prothésée ; et/ou
- plutôt qu'un même ancillaire intègre tous les éléments de guidage respectivement associés de manière univoque aux représentations graphiques à disposition, ce qui peut poser des problèmes d'encombrement lorsque ces éléments de guidage sont trop près les uns des autres, un jeu de plusieurs ancillaires est proposé, ces ancillaires étant pourvus de groupes respectifs d'éléments de guidage, chacun de ces groupes étant associé de manière univoque à un groupe correspondant de représentations graphiques ; l'identification du lien univoque entre l'un des ancillaires et l'un des groupes de représentations graphiques peut être réalisé, par exemple, à l'aide d'un marquage de couleurs.

## Revendications

1. Ensemble chirurgical d'assistance à l'implantation d'un composant glénoïdien de prothèse d'épaule sur un patient, comprenant un jeu d'au moins un ancillaire (10) pour appliquer un outil de préparation osseuse, tel qu'un foret de perçage, le ou chaque ancillaire comportant une platine (11), dont une face d'appui (16) est conformée de manière ajustée à la glène (G) du patient et qui est pourvue de moyens de guidage (12, 13, 14) adaptés pour guider l'application de l'outil sur la glène,
**caractérisé en ce que** l'ensemble comprend en outre au moins une série (20) de représentations graphiques de, à la fois, la glène (G) du patient et le composant glénoïdien (1) fictivement implanté sur cette glène, les positions respectives d'implantation du composant glénoïdien sur la glène pour ces représentations graphiques étant réparties selon un degré de liberté prédéfini pour la ou chaque série,
et **en ce que** les moyens de guidage (12, 13, 14) de la platine (11) sont adaptés pour guider l'application de l'outil sur la glène (G) selon plusieurs configurations dont chacune est associée de manière univoque à l'une des représentations graphiques.

2. Ensemble suivant la revendication 1, **caractérisé en ce que** chaque représentation graphique inclut un ou plusieurs dessins montrant la position correspondante d'implantation du composant glénoïdien (1) sur la glène (G) du patient.

3. Ensemble suivant l'une des revendications 1 ou 2, **caractérisé en ce que** chaque représentation graphique inclut au moins une valeur prédictivite représentative d'une caractéristique mécanique liée à l'implantation du composant glénoïdien (1) sur la glène (G) selon la position d'implantation montrée sur la représentation graphique.

4. Ensemble suivant l'une quelconque des revendications précédentes, **caractérisé en ce qu'**il comprend en outre des moyens d'obtention pré-opératoire des représentations graphiques, lesquels moyens d'obtention sont adaptés pour déterminer par calcul le positionnement relatif de la glène (G) et du composant glénoïdien (1) pour chaque représentation graphique, à partir de données de cartographie pré-opératoires relatives à la glène et de données dimensionnelles préétablies relatives au composant glénoïdien.

5. Ensemble suivant l'une quelconque des revendications précédentes, **caractérisé en ce que**, pour la ou chaque série, les positions respectives d'implantation fictive du composant glénoïdien (1) sur la glène (G) montrées sur les représentations graphiques sont réparties de manière prédéterminée, notamment avec un pas préfixé, selon le degré de liberté de la série.

6. Ensemble suivant l'une quelconque des revendications précédentes, **caractérisé en ce que** les représentations graphiques de la ou chaque série (20) sont ventilées en plusieurs groupes, et **en ce que** le jeu d'ancillaires inclut autant d'ancillaires (10) que de groupes précités, en étant respectivement associés de manière univoque à l'un de ces groupes.

7. Ensemble suivant l'une quelconque des revendications précédentes, **caractérisé en ce que** les représentations graphiques sont imprimées sur un support ou affichées sur un écran vidéo.

8. Ensemble suivant l'une quelconque des revendications précédentes, **caractérisé en ce que** les moyens de guidage de la platine (11) comportent des éléments fixes (12, 13, 14) de guidage de l'outil et/ou un organe mobile de guidage de l'outil, chacun de ces éléments de guidage fixes étant associé de manière univoque à l'une des représentations graphiques et/ou l'organe de guidage mobile étant déplaçable par rapport à la platine entre des positions de réglage dont chacune est associée de manière univoque à l'une des représentations graphiques.

9. Ensemble suivant l'une quelconque des revendications précédentes, **caractérisé en ce que** la conformation de la face d'appui (16) de la platine (11) est adaptée pour être personnalisée spécifiquement au patient pour n'autoriser qu'une seule configuration de coopération mécanique entre la platine et la glène (G) du patient.

10. Ensemble suivant l'une quelconque des revendications précédentes, **caractérisé en ce que** l'association univoque entre les configurations d'application fournies par les moyens de guidage (12, 13, 14) et les représentations graphiques de la ou chaque série (20) est réalisée par des éléments d'identification alphanumériques (« A », « B », « C »).

## Claims

1. Surgical assembly for assisting in the implanting of a glenoid component of a shoulder prosthesis in a patient, comprising a set of at least one ancillary (10) for applying a bone preparation tool, such as a drill bit, the or each ancillary comprising a plate (11), of which a bearing face (16) is conformed to be fitted to the socket (G) of the patient and which is provided with guidance means (12, 13, 14) suitable for guiding the application of the tool on the socket,
**characterized in that** the assembly also comprises at least one series (20) of graphic representations of, simultaneously, the socket (G) of the patient and the glenoid component (1) hypothetically implanted on this socket, the respective positions of implantation of the glenoid component on the socket for these graphic representations being distributed according to a degree of freedom predefined for the or each series,
and **in that** the guidance means (12, 13, 14) of the plate (11) are suitable for guiding the application of the tool on the socket (G) according to several configurations, each of which is associated on a one-to-one basis with one of the graphic representations.

2. Assembly according to claim 1, **characterized in that** each graphic representation includes one or more drawings showing the corresponding position of implantation of the glenoid component (1) on the socket (G) of the patient.

3. Assembly according to one of claims 1 or 2, **characterized in that** each graphic representation includes at least one predictive value representative of a mechanical characteristic associated with the implantation of the glenoid component (1) on the socket (G) according to the position of implantation shown on the graphic representation.

4. Assembly according to any one of the preceding claims, **characterized in that** it also comprises means of obtaining, pre-operation, graphic representations, which obtaining means are suitable for determining by calculation the relative positioning of the socket (G) and of the glenoid component (1) for each graphic representation, based on pre-operation mapping data relating to the socket and preestablished dimensional data relating to the glenoid component.

5. Assembly according to any one of the preceding claims, **characterized in that**, for the or each series, the respective positions of hypothetical implantation of the glenoid component (1) on the socket (G) shown on the graphic representations are distributed in a predetermined manner, notably with a preset pitch, according to the degree of freedom of the series.

6. Assembly according to any one of the preceding claims, **characterized in that** the graphic representations of the or each series (20) are broken down into a number of groups, and **in that** the set of ancillaries includes as many ancillaries (10) as there are abovementioned groups, while being respectively associated on a one-to-one basis with one of these groups.

7. Assembly according to any one of the preceding claims, **characterized in that** the graphic representations are printed on a medium or displayed on a video screen.

8. Assembly according to any one of the preceding claims, **characterized in that** the guidance means for the plate (11) include fixed elements (12, 13, 14) for guiding the tool and/or a mobile member for guiding the tool, each of these fixed guidance elements being associated on a one-to-one basis with one of the graphic representations and/or the mobile guidance member being removable relative to the plate between adjustment positions, each of which is associated on a one-to-one basis with one of the graphic representations.

9. Assembly according to any one of the preceding claims, **characterized in that** conformation of the bearing face (16) of the plate (11) is suitable for being customized specifically to the patient to allow only a single configuration of mechanical cooperation between the plate and the socket (G) of the patient.

10. Assembly according to any one of the preceding claims, **characterized in that** the one-to-one association between the application configurations supplied by the guidance means (12, 13, 14) and the graphic representations of the or each series (20) is produced by alphanumeric identification elements ("A", "B", "C").

## Patentansprüche

1. Chirurgische Anordnung zur Unterstützung bei der Implantierung einer Glenoidkomponente einer Schulterprothese bei einem Patienten, umfassend ein Set von mindestens einem chirurgischen Hilfsteil (10), um ein Instrument, wie einen Bohrer, zur Knochenbehandlung anzusetzen, wobei das oder jedes Hilfsteil eine Platte (11) aufweist, deren Auflagefläche (16) derart ausgebildet ist, dass sie an die Gelenkpfanne (G) eines Patienten angepasst ist und die mit Führungsmitteln (12, 13, 14) versehen ist, die dazu dienen, die Anwendung des Instruments an der Gelenkpfanne zu führen, **dadurch gekennzeichnet, dass** die Anordnung außerdem mindestens eine Reihe (20) von graphischen Darstellungen der Gelenkpfanne (G) des Patienten zusammen mit der Glenoidkomponente (1) umfasst, die fiktiv an dieser Gelenkpfanne implantiert ist, wobei die jeweiligen Positionen der Implantierung der Glenoidkomponente an der Gelenkpfanne für diese graphischen Darstellungen gemäß einem vorbestimmten Freiheitsgrad für die oder jede Reihe verteilt sind, und dass die Führungsmittel (12, 13, 14) der Platte (11) angepasst sind, um die Anwendung des Instruments an der Gelenkpfanne gemäß mehreren Konfigurationen zu führen, von denen jede in eindeutiger Weise einer der grafischen Darstellungen zugeordnet ist.

2. Anordnung nach Anspruch 1, **dadurch gekennzeichnet, dass** jede grafische Darstellung ein oder mehrere Zeichnungen einschließt, die die korrespondierende Position der Implantierung der Glenoidkomponente (1) an der Schulterpfanne (G) des Patienten zeigen.

3. Anordnung nach einem der Ansprüche 1 oder 2, **dadurch gekennzeichnet, dass** jede grafische Darstellung mindestens einen Eingabehilfswert einschließt, der repräsentativ für eine mechanische Eigenschaft ist, die mit der Implantierung der Glenoidkomponente (1) an der Gelenkpfanne (G) verknüpft ist, gemäß der auf der grafischen Darstellung gezeigten Position der Implantierung.

4. Anordnung nach einem beliebigen der vorhergehenden Ansprüche, **dadurch gekennzeichnet, dass** sie außerdem Mittel zum präoperativen Erhalten von grafischen Darstellungen umfasst, wobei die Mittel zum Erhalten angepasst sind, um durch Berechnung die relative Positionierung der Schulterpfanne (G) und der Glenoidkomponente (1) für jede grafische Darstellung aus den präoperativen Kartographiedaten bezüglich der Gelenkpfanne und aus den vorgegebenen Abmessungsdaten bezüglich der Gelenkkomponente zu bestimmen.

5. Anordnung nach einem beliebigen der vorhergehenden Ansprüche, **dadurch gekennzeichnet, dass** für die oder jede Reihe die jeweiligen Positionen der fiktiven Implantierung der Glenoidkomponente (1) an der Gelenkpfanne (G), die auf den grafischen Darstellungen gezeigt sind, in vorgegebener Weise, insbesondere mit einer festgelegten Schrittweite, gemäß dem Freiheitsgrad der Reihe verteilt sind.

6. Anordnung nach einem beliebigen der vorhergehenden Ansprüche, **dadurch gekennzeichnet, dass** die grafischen Darstellungen der oder jeder Reihe (20) in mehrere Gruppen aufgeteilt sind und dass das Set von chirurgischen Hilfsteilen so viele chirurgische Hilfsteile (10) wie vorerwähnte Gruppen einschließt, die jeweils in eindeutiger Weise einer dieser Gruppen zugeordnet sind.

7. Anordnung nach einem beliebigen der vorhergehenden Ansprüche, **dadurch gekennzeichnet, dass** die grafischen Darstellungen auf einen Träger gedruckt sind oder auf einem Videobildschirm angezeigt werden.

8. Anordnung nach einem beliebigen der vorhergehenden Ansprüche, **dadurch gekennzeichnet, dass** die Führungsmittel der Platte (11) feste Elemente (12, 13, 14) zur Führung des Instruments und/oder ein mobiles Organ zur Führung des Instruments aufweisen, wobei jedes dieser festen Führungselemente in eindeutiger Weise einer der grafischen Darstellungen zugeordnet sind und/oder das mobile Führungsorgan in Bezug auf die Platte zwischen Einstellpositionen bewegbar ist, von denen jede in eindeutiger Weise einer der grafischen Darstellungen zugeordnet ist.

9. Anordnung nach einem beliebigen der vorhergehenden Ansprüche, **dadurch gekennzeichnet, dass** die Gestaltung der Auflagefläche (16) der Platte (11) angepasst ist, um speziell für den Patienten personalisiert zu werden, um nur eine Konfiguration der mechanischen Zusammenarbeit zwischen der Platte und der Gelenkpfanne (G) des Patienten zuzulassen.

10. Anordnung nach einem beliebigen der vorhergehenden Ansprüche, **dadurch gekennzeichnet, dass** die eindeutige Zuordnung zwischen den Anwendungskonfigurationen, die von den Führungsmitteln (12, 13, 14) und den grafischen Darstellungen der oder jeder Reihe (2) geliefert werden, durch alphanumerische Identifikationselemente ("A", "B", "C") realisiert sind.
